# EUROPEAN PATENT APPLICATION

(11) **EP 4 374 789 A1**
(43) Date of publication of application: **29.05.2024**
(21) Application number: 22306621.8
(22) Date of filing: 26.10.2022
(51) Int. Cl.: A61B 5/145, A61B 5/1486, C25D 13/14, C25D 13/22, C25D 17/00, C25D 17/06, C25D 21/12

(54) **CLOSE FLOW CELL PROCESS FOR FABRICATING MICRONEEDLES**

(71) Applicant: PKvitality, 75001 Paris (FR)
(72) Inventor: PIERART, Luc, 94800 VILLEJUIF (FR); PEACOCK, Martin, ROYSTON, SG8 5HB (GB); TOWNSEND, Christopher, Mitcheldean, GL17 0D (GB)

(57) **Abstract**

The invention relates to A method comprising the steps of
a) disposing an array (10) of microneedles (11, 11', 11") in a tightly sealed enclosure (20), so that the microneedles (11, 11', 11") be in front of a conductive element (12) at equidistance of every microneedle (11, 11', 11") and not in contact with the tips of the microneedles (11, 11', 11");
b) injecting a first gas into the enclosure (20) for drying the microneedles (11, 11', 11");
c) injecting into the enclosure (20) a solution (13) comprising an active element (14) so that the microneedles (11, 11', 11") and the conductive element (12) be immersed into the solution (13);
d) applying a potential (V) or a current (I) between the conductive element (12), forming a cathode, and at least one microneedle (11, 11') of the array (10) of microneedles (11, 11', 11"), forming an anode, causing an electrodeposition of a uniform and homogenous film on the microneedles (11, 11'), the film comprising the active element (14).

## Description

### TECHNICAL FIELD

The present application relates to electrochemical sensors and in particular sensors for measuring a body analyte by means of microneedles. More precisely, the present application concerns a system and method for fabricating and/or functionalizing such microneedles for use in a device for measuring a body analyte.

### BACKGROUND OF THE INVENTION

Certain pathologies such as diabetes require daily monitoring of biochemical parameters of the human body, such as concentrations glucose containing in the interstitial liquid.

There are known devices with microneedles, which have the advantage of being less invasive that of conventional needles. Document WO 2020/025821 A1 discloses such a device.

These devices use amperometric measures for detecting the analyte so that the microneedles need to have chemical sensing capabilities. Indeed, for the detection of the glucose, the microneedles have an enzyme deposited at the tips of the microneedles.

For the deposition of an element such as metal particle or an enzyme, it is possible to realize electrodeposition and/or polymerization for entrapping metal particle and/or enzyme onto the surface of the microneedles.

However, this fabrication of the microneedles is not homogenous especially when a large number of microneedles need when need to be manufactured.

### SUMMARY OF THE INVENTION

The present invention aims at improving the fabrication of microneedles for use in a device for measuring a body analyte such as glucose, lactate etc.

To this end, the invention concerns according to a first aspect a method comprising the steps of:
a) disposing an array of microneedles in a tightly sealed enclosure, so that the microneedles be in front of a conductive element at equidistance of every microneedle and not in contact with the tips of the microneedles,
b) injecting a first gas into the enclosure for drying the microneedles;
c) injecting into the enclosure a solution comprising an active element so that the microneedles and the conductive element be immersed into the solution,
d) applying a potential or a current between the conductive element, forming a cathode, and at least one microneedle of the array of microneedles, forming an anode, causing an electrodeposition of a uniform and homogenous film on the microneedles, the film comprising the active element

The invention according to the first aspect may comprise at least one of the following features, alone or in combination:
- it comprises a cleaning of the tightly sealed enclosure comprising the sub steps of:
   e) removing the solution from the tightly sealed enclosure; f) injecting into the tightly sealed enclosure a cleaning liquid for cleaning the enclosure g) removing the cleaning liquid, by preferably injecting a second gas into the tightly sealed enclosure.
- the step of injecting the first gas into the tightly sealed enclosure further removes the oxygen from the tightly sealed enclosure;
- an external reference electrode is disposed into the tightly sealed enclosure not to be in contact with the microneedles and the conductive element, and not between the microneedles and the conductive element, the potential or current being also applied to the external reference electrode.
- the conductive element is a conductive grid or mesh plate or array of tips.
- the solution is a saline solution, the active element being conductive particles or an electroactive monomer precursor with a biochemical element.
- the tip of each microneedle is in front of the conductive element, each at a same distance of the conductive element, the distance being comprised between 0,1mm and 10mm.
- it comprises a testing step for testing the metallized microneedles or functionalized microneedles, the method comprising the steps of: h) injecting into the tightly sealed enclosure a testing solution so that the microneedles and the conductive element be immersed into the testing solution; i) applying a potential or a current (between the conductive element, forming a cathode, and at least one metallized microneedle or functionalized microneedle forming an anode; j) measuring during step i) a resistance between the microneedles and the conductive element; k) comparing the measured resistance to a golden resistance value characterizing a quality of the microneedles.
- the testing solution comprises sodium.
- the potential or current is an oxidizing potential or current and/or a reducing potential or current, the potential or current being a fixed potential or a variable potential said potential or current being applied using amperometric method and/or chronoamperometric method and/or potentiometric method and/or chronometric method and/or voltametric cycle.

The invention according to the first aspect may be used as a metallization step wherein the active element is conductive particles, the microneedles obtained at step d) are metallized microneedles adapted to be an electrode for an electrochemical sensor.

The invention according to the first aspect may be used as a functionalizing step wherein the active element is an electroactive monomer precursor with a biochemical element, the microneedles obtained at step d) are functionalized microneedles adapted to be used in a working electrode for an electrochemical sensor.

According to an embodiment, the metallization step and the functionalization step and eventually the testing step are implemented successively without unsealing the enclosure and removing the array of microneedles from the system and by only implementing a cleaning step comprising the steps f) to h) before each of the metallization step, the functionalization step and the testing step.

The invention according to a second aspect concerns a system comprising:
an enclosure configured to be filled with a solution comprising an active element,
a cover configured to seal tightly the enclosure;
at least one conductive element intended to be disposed in front of an array of microneedles and at equidistance of every microneedle and not in contact with the tips of the microneedles;
a potential or current source configured to apply a potential or an current between the conductive element, forming a cathode, and at least one microneedle, forming an anode, the conductive element aiming at an electrodeposition of a uniform and homogenous film on the microneedles when immersed in the solution and when the potential or the current is applied between at least one microneedle and, the film comprising the active element.

The invention according to the second aspect may comprise one of the following features alone or in combination:
- it comprises a first inlet adapted to inject a first gas into the enclosure and a first outlet adapted to eject the first gas from the enclosure, the system comprising a first channel between the first inlet and the first outlet for the circulation of the first gas into the system.
- it comprises a second inlet adapted to inject into the enclosure a solution and a second outlet adapted to remove a solution from the enclosure, the system comprising a second channel between the second inlet and the second outlet for the circulation of the solution into the system.

- it comprises a third inlet adapted to inject into the enclosure a cleaning liquid and a third outlet to eject the cleaning liquid, the system comprising a third channel between the third inlet and the third outlet for the circulation cleaning liquid into the system.
- it comprises a fourth inlet adapted to inject a second gas into the enclosure and a fourth outlet adapted to eject the second gas from the enclosure, the system comprising a fourth channel between the fourth inlet and the fourth outlet for the circulation of the second gas into the system.
- it comprises opening and closing means at each inlet and outlet of each channel.

The invention has several advantages.

The use of a conductive element well-disposed allows a deposit of a homogenous conductive layer or enzyme layer.

Furthermore, this process is suitable for a scale-up manufacturing.

Moreover, the use of a seal enclosure allows to control the atmosphere in which the deposit is made. In particular, it is possible to remove the oxygen from the enclosure improving the fabrication.

Also, by injecting the solution in the enclosure, it is possible to control the volume injected so only an amount required for the reaction is used.

The channels allow to choose the different flows and to control them.

The close flow cell improves quality and the productivity of the fabrication.

According to an embodiment, the application of the potential between the cathode and the anode aims at: an electrodeposition when the active element comprised in the solution is metal particles or enzyme; measuring a resistance when the active element comprised in the solution is sodium.

### BRIEF DESCRIPTION OF THE DRAWINGS

Other features and advantages of the invention will appear in the following description. Embodiments of the invention will be described with reference to the drawings, in which:
- Figure 1 illustrates a view of the different elements of a body monitoring device using microneedles fabricated with the invention;
- Figure 2 illustrates a schematic view of a system for fabricating microneedles according to the invention;
- Figure 3 and 4 illustrate different steps of the method of the invention;
- Figure 5 illustrates an embodiment of a system for fabricating microneedles, according to the invention.

On all the figures, similar elements have the identical references.

### DETAILED DESCRIPTION OF THE INVENTION

### Body monitoring device with sensors

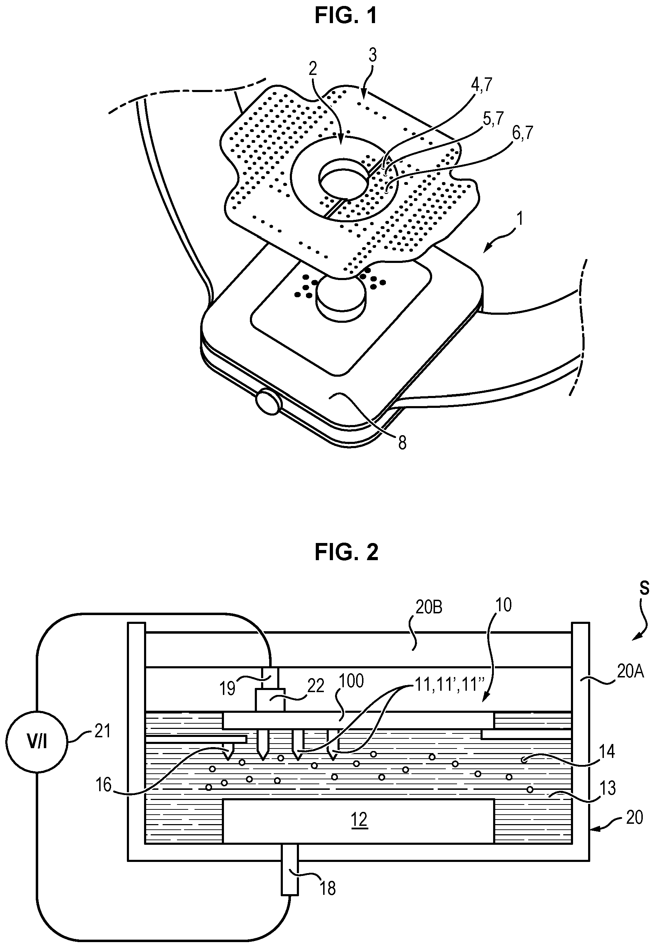

**Figure 1** illustrates a device 1 for measuring/monitoring a body analyte in fluid of a user comprising a sensor 2 surrounded by a patch 3 including an adhesive layer for attaching the sensor 2 to the wearer's skin.

The sensor 2 comprises a working electrode 4, a counter electrode 5 and preferably a reference electrode 6. These electrodes are inserted in the dermis of a user by several microneedles 7 to as to be in contact with the interstitial fluid of the user. Each electrode is thus constituted by at least one microneedle 7. The sensor 2 is in particular an electrochemical sensor.

The sensor 2 preferably comprises between four and fifty microneedles 7 or even four hundred microneedles 7. Of course, a different number may be considered without limiting the scope of the description.

A microneedle 7 is defined as a needle with a low height preferably between 0.2 µm and 1 mm, preferably between 0.3 mm and 0.8 mm. The height of the microneedles 7 is low enough to avoid contact with a mechanical pain nerve of the wearer when the device 1 is worn.

The microneedles 7 are adapted to pierce the skin to contact the interstitial fluid when the sensor 2 is brought into contact with the skin.

The device 1 also comprises a control unit 8. The control unit 8 is electrically connected to the electrodes, particularly to the working electrode 4, to the counter electrode 5, and preferably to the reference electrode 6. The control unit 8 can comprise processor, a memory, an electrical acquisition module and an electrical control module. Preferably, every working electrode 4 and counter electrode 5 is independently electrically connected to the electrical acquisition module and to the electrical control module.

Each microneedle 7 comprises on its surface at least one electrically conductive layer and a biochemical element (such an enzyme) for the working electrode, the biochemical element being configured for oxidizing the analyte.

The conductive layer is for example platinum or silver or gold etc. The enzyme comprises a cofactor which is responsible for the oxidation of the analyte, preferably glucose or lactate. The enzyme is preferably an enzyme chosen between glucose oxidase and dehydrogenase, such as PQQ-glucose dehydrogenase, NAD-glucose dehydrogenase or FAD-glucose dehydrogenase or lactate oxidase, etc.

The electrically conductive layer is obtained during a metallization step and the enzyme is deposited during a functionalization step of a method for fabricating microneedle as it will be described in the following. The functionalization is in particular performed using electrodeposition concept.

### Metallization and/or functionalization of an array of microneedle

In the following, we describe metallization and functionalization implemented for the fabrication of microneedles 7 intended to be used into an electrochemical sensor 2 as, for instance, described above.

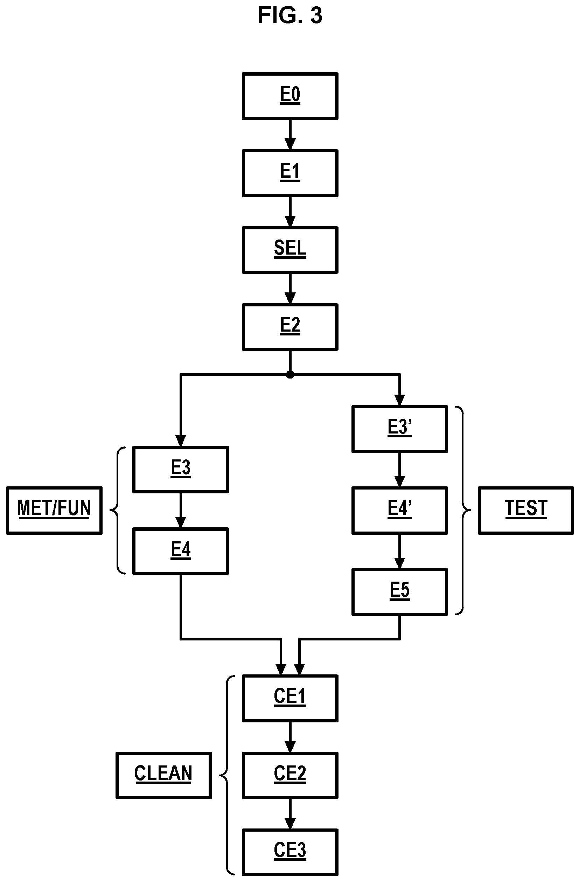

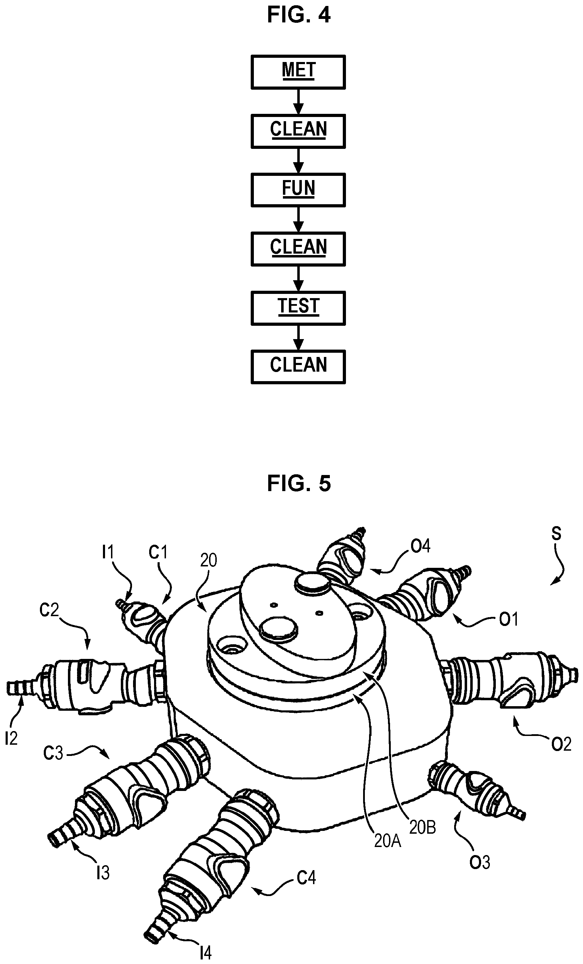

**Figure 2** illustrates a schematic system S for implementing the metallization and/or functionalization of raw microneedles 11 according to a first embodiment, **figures 3** and 4 illustrate steps of a method implemented in the system of figure 2.

In the following:
- by "microneedle" 7 is meant a microneedle usable to be used on a sensor 2, and by "raw microneedle" 11, a microneedle which is a microneedle needing treatments in order to be ready to be used in the electromechanical sensor 2;
- by "metallized microneedle 11'" is meant a raw microneedle 11 that has been metallized;
- by "functionalized microneedle 11"" is meant a metallized microneedle 11' that has been functionalized

A raw microneedle 11, a metallized microneedle 11' and a functionalized microneedle 11" have therefore a shape identical to the microneedle 7 used in the electromechanical sensor 2. For the sake of clarity, we will use microneedle for designating any type of microneedle and we will precise the type in view of the context if needed.

The system S comprises a conductive element 12, an enclosure 20, in which the conductive element 12 and at least one microneedle 11, 11', 11", preferably in a form of array is disposed. The enclosure 20 is defined by several walls 20A and may have several shapes: circular, rectangular, etc.

The enclosure 20 is configured to comprise a solution 13 for immersing the microneedles 11, 11', 11" and the conductive element 12.

In order to seal the enclosure 20, the system S comprises a cover 20B. The cover 20B is in particular configured to tightly seal the enclosure 20.

Preferably and as illustrated, the array 10 of microneedles 11, 11', 11" is disposed in front of the conductive element 12 disposed into the enclosure 20. The conductive element 12 is, in particular, at equidistance of every microneedle 11, 11', 11" and not in contact with the tips of the microneedles 11, 11', 11". Preferably, the tips of each raw microneedle 11, 11', 11" is in front of the conductive element 12, each at a same distance of the conductive element 12, the distance being, for instance, comprises between 0,1 mm and 10 mm. The positioning accuracy must be greater than 90%, i.e., the distance between each tip of microneedle and the conductive element 12 must not be greater than 10% of this distance.

The conductive element 12 is preferably a grid with the axis of the holes parallel to the array 10 of raw microneedles 11. Optionally the conductive element 12 is a plate of any form or it can be a network of tips positioned in front of each raw microneedle 11. By tips it is meant everything that can protrude regularly or not from a surface and that extends in the same direction i.e., bosses, points, crenellations, curves.

The conductive element 12 and the array of microneedles 11, 11', 11" are connected to a power source 21 configured to supply a potential V or a current I to the conductive element 12 and the array of microneedles 11, 11', 11".

Once the array 10 of microneedles 11, 11', 11" is disposed into the enclosure 20 (step E0), the enclosure 20 is tightly sealed (step E1). The sealing of the enclosure 20 is obtained by the cover 20B and permits to control the atmosphere into the enclosure 20.

After the sealing, a first gas is injected into the enclosure 20 (step E2). This first gas permits to remove or to replace the oxygen presents in the enclosure so that the atmosphere is controlled and adapted to the following steps for the deposition of an active element as it will be described. Furthermore, the first gas permits to dry the microneedles 11, 11', 11". The first gas is for instance nitrogen or argon.

A solution 13 comprising an active element 14 is then injected into the enclosure 20 so that the microneedles 11, 11', 11" and the conductive element 12 be immersed into the solution 13 (step E3).

Advantageously, the solution 13 is a phosphate-buffered saline solution. The active element 14 comprised in the solution 13 is metallic particles or an electroactive monomer precursor. The solution 13 with metallic particles is used for a metallization step (MET) and the solution 13 with the enzyme is used for the functionalization step (FUN). The metallic particles are for instance made platinum or silver or gold, etc. The electroactive monomer precursor is for instance, Pyrrole, with a biochemical element 9, e.g., GOX or another enzyme as already described.

Then a potential V or a current I between the conductive element 12, forming a cathode, and at least one microneedle 11, 11' of the array 10, forming an anode, is applied (step E4), causing an electrodeposition of a uniform and homogenous film on the microneedles 11, 11', the film comprising the active element 14. The electrodeposition is thus implemented in a controlled atmosphere. The anode is defined by each microneedle 11, 11', 11" connected to the potential or current source 21, each microneedle 11, 11', 11", being selected (step SEL) before the application of the potential V. Preferably, the potential V (or current I) applied is an oxidizing potential V (or current I) and/or a reducing potential V (or current I), the potential V (or current I) being a fixed potential V (or a current I) or a variable potential V (or a current I) said potential (V) or current (I) being applied (step E2) using amperometric method and/or chronoamperometric method and/or potentiometric method and/or chronometric method and/or voltametric cycle. The potential or current is applied during a time that can be from a few minutes to a few hours. The amplitude of the potential V which is applied can be from a few ten millivolt to a few hundred millivolt.

The cathode and the anode are subject to the potential V or the current I to start electrodeposition. The electrodeposition applies a uniform and homogenous film on the microneedles 11, 11' representing the anode, i.e., on which the potential V or the current I is applied. During the electrodeposition the film comes from active element 14 comprised in the solution 13.

The microneedles 11', 11" obtained with the method herein described can be used as different type of electrode and during the fabrication it is possible to select a group of microneedles 11, 11' and to use the adequate solution 13, and then to apply the potential V or the current only on the selected microneedles 11, 11'. It has to be understood that only the microneedles 11, 11', 11" on which the potential V or current is applied are metallized or functionalized or tested (see below).

Advantageously, in addition to the conductive element 12, an external reference electrode 16 is located into the enclosure 20, therefore immersed, and the potential V or current is also applied to this external reference electrode 16. This external reference electrode 16 is in particular disposed not to be in contact with the microneedles 11, 11', 11" and the conductive element 12 and not between the microneedles 11, 11', 11" and the conductive element 12. The external reference electrode 16 is used to control and/or adapt the amplitude and/or the duration of the potential V or the current I. The conductive element 12 and the external reference electrode 16 are always subjected to the potential V or current I when present.

As illustrated on figure 2, the system S comprises a first electrical connector 18 to connect the conductive element 12 to the potential or current source 21, a second electrical connector 19 connected to the array 10 of microneedles 11, 11', 11" to the potential or current source 21. If needed, the external reference electrode 16 is also connected to the potential or current source 21.

Advantageously, the system S includes a seal 22 to isolate the electrical connector 19 from the solution 13 when the array 10 of microneedles 11, 11', 11" is immersed. The array 10 of microneedles 11, 11', 11" comprises a plate 100 from which the microneedles extend.

The array 10 of microneedles is immersed so that the microneedles 11, 11', 11", are parallel to the direction of immersion and the electrical connector 19 stays out of the solution 13 and is isolated from it by the seal 22.

As illustrated in figure 3, once the array 10 of microneedles 11, 11', is disposed in front of the conductive element 12 (step E0) and the enclosure 20 sealed (step E1), the method may comprise a selection of a group of at least one microneedle 11, or metallized microneedle 11' (step SEL) for defining an anode and then comprises the steps E2, E3 and E4. Steps E2, E3 and E4 define a metallization step (step MET) when the solution comprises metallic particles and define a functionalization step (step FUN) when the solution comprises the enzyme.

It can be noticed that the functionalizing step (step FUN) is preferentially processed on microneedles 11 which have been metallized before, i.e. metallized microneedles 11'.

The metallization and the functionalization can be implemented successively without having to unseal the enclosure. Each deposit is performed on the microneedles 11, 11' which are connected to the potential or current source 21. Preferably, it is possible to connect independently each microneedle 11, 11', 11", or group of microneedles 11, 11', 11", to the potential or current source 21, and then to turn on power for only some microneedles 11, 11', 11", of the array 10 of microneedles 11, 11', 11".

As illustrated on figure 3, after the metallization and functionalization steps, the method may comprise a step of cleaning the enclosure 20 (step CLEAN). The cleaning step comprises the subs steps of removing the solution 13 from the enclosure 20 (step CE1); injecting into the enclosure 20 a cleaning liquid for cleaning the enclosure (20) (step CE2); removing the cleaning liquid by injecting a cleaning gas (step CE3). The cleaning liquid is for example water. The cleaning permits to further use another solution without the contamination of the other solution. The second gas is advantageously air.

Additionally, the sealed enclosure 20 can be used for testing the metallized and/or functionalized microneedles. Thus, the method comprises a testing step (step TEST) using a solution comprising a testing element 14 such as sodium.

Here again, before and after the testing step, the cleaning step (step CLEAN) is implemented.

The testing step (step TEST) comprises the sub steps injecting (step E3) a testing solution 13' comprising sodium into the sealed enclosure 20 and applying (step E4') a potential V or a current I for measuring (step E5) the resistance of the anode and thus, testing the quality of the obtained microneedles 11', 11". In particular, the measured resistance is compared (step E6) to a golden resistance value which is a reference value and a target for the metallized and/or functionalized microneedles. The golden resistance has been preferably obtained with a dedicated process especially implemented to obtain the golden resistance values corresponding to expected values. The testing solution 13' is also chosen to correspond to the conditions for which the golden resistance values have been obtain.

The testing step (step TEST) allows to measure the efficiency of the preceding steps as metallization step (step MET) and/or the functionalization step (step FUN).

As illustrated by figure 5, the system S preferentially comprises a plurality of channels C1, C2, C3, C4 for the circulation of the different gas and/or liquid into the enclosure 20. Each channel C1, C2, C3, C4 comprises an inlet and an outlet.

Advantageously, the system S comprises:
- a first inlet I1 adapted to inject the first gas into the enclosure 20 and a first outlet O1 adapted to eject the first gas from the enclosure 20,
- a second inlet I2 adapted to inject into the enclosure 20 the solution 13 and a second outlet O2 adapted to eject the solution 13 from the enclosure 20,
- a third inlet I3 adapted to inject into the enclosure 20 the cleaning liquid and a third outlet O3 to eject the cleaning liquid, and
- a fourth inlet I4 adapted to inject the second gas into the enclosure 20 and a fourth outlet O4 adapted to eject the second gas from the enclosure 20.

Each inlet and outlet comprise opening and closing means to control the different flows and the proper functioning of the system S. Each channel C1, C2, C3, C4 is used for the circulation of its own gas or liquid. It is optionally possible to add mor channels, so more inlet and outlet, if necessary. These channels C1, C2, C3, C4 allow to use system S comprising a sealed enclosure 20 while controlling the different flows and concentration into the enclosure 20. For example, by using a sealed enclosure 20 and the first channel C1 it is possible to regulate the oxygen concentration before the injection of the solution 13 into the enclosure 20. The metallization and the functionalization can be implemented successively without removing the array 10 of different types of microneedles 11, 11', 11" from the enclosure 20. Each deposit is performed on the microneedles 11, 11', which are connected to the potential or current source 21. Preferably, it is possible to connect independently each microneedle 11, 11', or group of microneedles 11, 11', to the potential V or current I, and then to disconnect the potential V or current I and apply it again for some others microneedles 11, 11'.

As illustrated on figure 4, the metallization step (step MET) and/or the functionalization step (step FUN) and the testing step (step TEST) are implemented successively without unsealing the enclosure 20 and removing the array 10 of microneedles from the system S and by only realizing the cleaning step (step CLEAN) between each of these steps.

In a complementary way, the system S can be used to fabricate several arrays 10 at the same time by disposing several arrays 10 of microneedles 11, 11', 11", into the enclosure 20, above the conductive element 12.

## Claims

1. A method comprising the steps of
a) disposing (E0, E1) an array (10) of microneedles (11, 11', 11") in a tightly sealed enclosure (20), so that the microneedles (11, 11', 11") be in front of a conductive element (12) at equidistance of every microneedle (11, 11', 11") and not in contact with the tips of the microneedles (11, 11', 11"),
b) injecting (E2) a first gas into the enclosure (20) for drying the microneedles (11, 11', 11");
c) injecting (E3) into the enclosure (20) a solution (13) comprising an active element (14) so that the microneedles (11, 11', 11") and the conductive element (12) be immersed into the solution (13),
d) applying (E4) a potential (V) or a current (I) between the conductive element (12), forming a cathode, and at least one microneedle (11, 11') of the array (10) of microneedles (11, 11', 11"), forming an anode, causing an electrodeposition of a uniform and homogenous film on the microneedles (11, 11'), the film comprising the active element (14).

2. The method as claimed in claim 1, comprising a cleaning (CLEAN) of the tightly sealed enclosure (20) comprising the sub steps of:
e) removing the solution (13) from the tightly sealed enclosure (20);
f) injecting into the tightly sealed enclosure (20) a cleaning liquid (20) for cleaning the enclosure (20);
g) removing the cleaning liquid, by preferably injecting a second gas into the tightly sealed enclosure (20).

3. The method as claimed in any one of claims 1 to 2, wherein the step of injecting the first gas into the tightly sealed enclosure further removes the oxygen from the tightly sealed enclosure (20).

4. The method as claimed in any one claims 1 to 3, wherein an external reference electrode (16) is disposed into the tightly sealed enclosure (20) not to be in contact with the microneedles (11, 11', 11") and the conductive element (12), and not between the microneedles (11, 11', 11") and the conductive element (12), the potential (V) or current (I) being also applied to the external reference electrode (16).

5. The method as claimed in any one of claims 1 to 4, wherein the conductive element (12) is a conductive grid or mesh plate or array of tips.

6. The method as claimed in any one of claims 1 to 5, wherein the solution (13) is a saline solution, the active element (14) being conductive particles or an electroactive monomer precursor with a biochemical element.

7. The method as claimed in any one of claims 1 to 6, wherein the tip of each microneedle (11, 11', 11") is in front of the conductive element (12), each at a same distance of the conductive element (12), the distance being comprised between 0,1mm and 10mm.

8. The method as claimed in any one of claims 1 to 7, for use as a metallization step (MET) wherein the active element (14) is conductive particles, the microneedles obtained at step d) are metallized microneedles (11') adapted to be an electrode (7) for an electrochemical sensor (2).

9. The method as claimed in any one of claims 1 to 8, for use as a functionalizing step (FUN) wherein the active element (14) is an electroactive monomer precursor with a biochemical element, the microneedles obtained at step d) are functionalized microneedles (11") adapted to be used in a working electrode (4) for an electrochemical sensor (2).

10. The method as claims in any one of claims 2 to 9, comprising a testing step (TEST) for testing the metallized microneedles (11') or functionalized microneedles (11"), the method comprising the steps of:
h) injecting (E3') into the tightly sealed enclosure (20) a testing solution (13') so that the microneedles (11, 11', 11") and the conductive element (12) be immersed into the testing solution,
i) applying a potential (V) or a current (I) (E4') between the conductive element (12), forming a cathode, and at least one metallized microneedle (11') or functionalized microneedle (11") forming an anode,
j) measuring (E5) during step i) a resistance between the microneedles (11', 11") and the conductive element (12),
k) comparing (E6) the measured resistance to a golden resistance value characterizing a quality of the microneedles.

11. The method as claimed in claim 10, wherein the testing solution (13') comprises sodium.

12. The method as claimed in any one of claims 10 to 11, wherein the metallization step (MET) and the functionalization step (FUN) and eventually the testing step (TEST) are implemented successively without unsealing the enclosure (20) and removing the array (10) of microneedles (11, 11', 11") from the system (S) and by only implementing a cleaning step (CLEAN) comprising the steps f) to h) before each of the metallization step (MET), the functionalization step (FUN) and the testing step (TEST).

13. Method according to any one of claims 1 to 12, wherein the potential (V) or current (I) is an oxidizing potential or current and/or a reducing potential or current, the potential (V) or current being a fixed potential or a variable potential said potential (V) or current (I) being applied (E2) using amperometric method and/or chronoamperometric method and/or potentiometric method and/or chronometric method and/or voltametric cycle.

14. System (S) comprising:
an enclosure (20) configured to be filled with a solution (8) comprising an active element (14),
a cover (20B) configured to seal tightly the enclosure (20);
at least one conductive element (12) intended to be disposed in front of an array (10) of microneedles (11, 11', 11") and at equidistance of every microneedle (11, 11', 11") and not in contact with the tips of the microneedles (11, 11', 11");
a potential or current source (21) configured to apply (E4) a potential (V) or an current (I) between the conductive element (12), forming a cathode, and at least one microneedle (11, 11', 11"), forming an anode, the conductive element (12) aiming at an electrodeposition of a uniform and homogenous film on the microneedles (11, 11') when immersed in the solution (13) and when the potential (V) or the current (I) is applied between at least one microneedle (11, 11') and, the film comprising the active element (14).

15. System according to the claim 14, comprising a first inlet (I1) adapted to inject a first gas into the enclosure (20) and a first outlet (O1) adapted to eject the first gas from the enclosure (20), the system comprising a first channel (C1) between the first inlet (I1) and the first outlet (O1) for the circulation of the first gas into the system (S).
